# EUROPÄISCHE PATENTSCHRIFT

(11) **EP 0 452 341 B1**
(45) Veröffentlichungstag und Bekanntmachung des Hinweises auf die Patenterteilung: **02.06.1993**
(21) Anmeldenummer: 90900113.3
(22) Anmeldetag: 28.11.1989
(51) Int. Cl.: A61L 2/06

(54) **STERILISIERBEHÄLTER**
STERILIZING CONTAINER
RECIPIENT STERILISATEUR

(30) Priorität: 03.01.1989 DE 3900049
(43) Veröffentlichungstag der Anmeldung: 23.10.1991
(73) Patentinhaber: AESCULAP AG, D-78501 Tuttlingen (DE)
(72) Erfinder: TASCHNER, Wolfgang, D-7200 Tuttlingen (DE); WÖLFLE, Wilfried, D-7737 Bad Dürrheim 7 (DE)
(74) Vertreter: Hoeger, Stellrecht & Partner
(86) Internationale Anmeldenummer: EP8901443
(87) Internationale Veröffentlichungsnummer: WO9007346

(56) Entgegenhaltungen:
- DE-C- 3 438 463
- DE-C- 3 838 099

## Beschreibung

Die Erfindung betrifft einen Sterilisierbehälter mit einem perforierten Bereich mit den Merkmalen, die im Oberbegriff des Patentanspruches 1 angegeben sind.

Derartige Sterilisierbehälter sind bereits vorgeschlagen worden (Deutsche Patentschrift 38 38 099).

Mit diesen speziell konstruierten Filtern ist es möglich, normalerweise den perforierten Bereich in der Wand oder im Deckel eines Sterilisierbehälters mittels der Filterlage abzudecken, so daß ein Gasaustausch durch die Perforation und die Filterschicht erfolgen kann. Sobald Gas mit hoher Geschwindigkeit in den Behälter eintritt, beispielsweise beim Einströmen des Wasserdampfes zum Sterilisieren, verschiebt dieser Dampf die Abdeckscheibe in Richtung auf das Filter, welches dadurch auf seiner Oberseite abgedeckt wird. Gleichzeitig verschiebt die abgedichtet am Filter anliegende Abdeckscheibe auch das Filter und legt dieses abgedichtet an die untere Abdeckung an. Dies führt dazu, daß das Filter auf beiden Seiten abgedichtet abgedeckt und somit vollständig geschützt ist. Durch die Verschiebung des Filters selbst wird die Abdichtung des Filters gegenüber dem perforierten Bereich der Behälterwand oder im Behälterdeckel entfernt, so daß nunmehr der Gasaustausch außerhalb der abgedichteten Bereiche erfolgen kann, ohne daß dabei eine Filterung eintritt. In dieser zweiten Betriebphase ist also die Baueinheit aus Abdeckscheibe, Filter und unterer Abdeckung aus dem eigentlichen Strömungsweg entfernt, während in der ersten Betriebphase das Filter im Strömungpfad liegt und beidseitig von Abdeckscheibe beziehungsweise unterer Abdeckung freigegeben wird. Auf diese Weise wird das Filter nur in einem Teil des Betriebsablaufes in den Strömungspfad eingeschaltet, während in einer anderen Betriebsphase, in der eine besonders starke Verschmutzung des Filters zu befürchten wäre, sich das Filter nicht im Strömungpfad befindet. Dies erlaubt eine wesentlich längere Verwendung eines bestimmten Filters bei Aufrechterhaltung derselben Betriebssicherheit.

Es ist Aufgabe der Erfindung, eine solche Filterkonstruktion noch dahingehend zu verbessern, daß beim normalen Umgang mit dem Sterilisierbehälter der Gasaustausch mit besonders großer Zuverlässigkeit über das Filter erfolgt, während beim Sterilisieren andererseits sichergestellt ist, daß das Filter zuverlässig von Abdeckung und Abdeckscheibe geschützt wird und anderseits der Bypaß-Strömungpfad, der am Filter vorbei ins Behälterinnere führt, wirksam freigegeben wird.

Diese Aufgabe wird bei einem Sterilisierbehälter der eingangs beschriebenen Art erfindungsgemäß dadurch gelöst, daß die die Abdeckscheibe und das Filter mit Verschiebekräften beaufschlagenden Kraftglieder, die auf die Abdeckscheibe beziehungsweise das Filter ausgeübten Verschiebekräfte in Abhängigkeit von der Temperatur ändern.

Die Verschiebung der Abdeckscheibe und des Filters erfolgt also nicht nur unter der Wirkung der Federn und der durch das einströmende Gas oder den einströmenden Dampf ausgeübten Druckstöße, sondern zusätzlich auch unter dem Einfluß der sich beim Sterilisiervorgang ändernden Temperatur, die damit die Freigabe beziehungsweise Umhüllung des Filters zusätzlich unterstützt.

Es kann dabei insbesondere vorgesehen sein, daß die Abdeckscheibe gegen das Filter drückende Kraftglieder die Andruckkräfte mit steigender Temperatur erhöhen. Weiterhin können die das Filter gegen die Abdeckung drückenden Kraftglieder so ausgebildet sein, daß sie die Andruckkräfte mit steigender Temperatur herabsetzen. Dies führt dazu, daß bei normaler Umgebungstemperatur die Abdeckscheibe vom Filter leichter abgehoben wird als bei hohen Temperaturen, bei denen sterilisiert wird. Außerdem wird das Filter bei tieferen Tempera- turen in seiner Anlage an die Bypaßöffnungen verschließenden Dichtungen unterstützt, so daß die Zuverlässigkeit dieser Dichtung erhöht und sichergestellt ist, daß bei tieferen Temperaturen eine Strömungsverbindung in jedem Fall nur über das Filter möglich ist.

Bei höheren Temperaturen, also während des Sterlisiervorganges, unterstützen aber die temperaturabhängigen Kraftglieder die Öffnung des Bypaß-Strömungsweges und die schützende Umhüllung des Filters, so daß die Öffnung des Bypaß-Strömungspfades und der Einschluß des Filters nicht allein durch die Druckstöße des einströmenden Dampfes erfolgen, sondern bereits entweder allein aufgrund der erhöhten Temperatur oder aber bei erhöhter Temperatur und relativ geringem Druckanstieg durch einströmenden Dampf.

Es kann vorgesehen sein, daß die Kraftglieder abgeschlossene Körper mit verformbaren Wänden sind, die ein Medium enthalten, das sich mit steigender Temperatur ausdehnt. So können die Körper mit einer Flüssigkeit gefüllt sein, die bei zunehmender Temperatur verdampft.

Derartige Körper können beispielsweise kissenförmig oder balgförmig ausgebildet sein.

Bei einem abgewandelten Ausführungsieispiel ist vorgesehen, daß die Kraftglieder aus Memory-Metall bestehen, so daß bei unterschiedlichen Temperaturen die Kraftglieder unterschiedliche Formgebung annehmen und damit in unterschiedlicher Weise Abdeckscheibe und/oder Filter mit Andruckkräften beaufschlagen. Es ist auch möglich, die Kraftglieder als Bimetall-Elemente auszubilden.

Günstig ist es, wenn ein Kraftglied zwischen dem perforierten Bereich und der Abdeckscheibe angeordnet ist, dieses Kraftglied unterstützt die Anlage der Abdeckscheibe am Filter und wirkt normalerweise der Feder entgegen, die die Abdeckscheibe vom Filter abhebt. Dabei ist diese Gegenwirkung zur Federkraft temperaturabhängig, so daß die Federkraft temperaturabhängig unterschiedlich kompensiert wird.

Günstig ist es weiterhin, wenn ein Kraftglied zwischen Abdeckung und Filter angeordnet ist, wobei dessen Wirkung umgekehrt ist wie die Wirkung eines Kraftgliedes zwischen dem perforierten Bereich und der Abdeckscheibe, das heißt dieses Kraftglied zwischen Abdeckung und Filter unterstützt bei niedrigen Temperaturen die Wirkung der Feder, die das Filter von der Abdeckung abhebt, bei höheren Temperaturen hingegen wird dieser unterstützende Anteil vermindert. Dabei ist es vorteilhaft, wenn sich die Feder zwischen Abdeckung und Filter an dem Kraftglied abstützt.

Die nachfolgende Beschreibung bevorzugter Ausführungsformen der Erfindung dient im Zusammenhang mit der Zeichnung der näheren Erläuterung. Es zeigen:
- Figur 1:: eine Querschnittsansicht eines Filterventils mit in den Strömungspfad eingeschaltetem Filter und mit einem balgförmigen Kraftglied zwischen perforiertem Bereich und Abdeckscheibe bei verschlossenem Bypaß-Strömungspfad;
- Figur 2:: eine Ansicht ähnlich Figur 1 bei geöffnetem Bypaß-Strömungspfad;
- Figur 3:: eine abgewandelte Ausführungsform mit einem Kraftglied aus Memory-Metall bei verschlossenem Bypaß-Strömungspfad;
- Figur 4:: eine Ansicht ähnlich Figur 3 bei geöffnetem Bypaß-Strömungspfad;
- Figur 5:: eine Ansicht ähnlich Figur 3 bei einem Ausführungsbeispiel mit einem kissenförmigen Kraftglied bei verschlossenem Bypaß-Strömungspfad;
- Figur 6:: eine schematische Ansicht ähnlich Figur 5 bei geöffnetem Bypaß-Strömungspfad;
- Figur 7:: ein abgewandeltes Ausführundgsbeispiel eines Filterventils mit zwei Kraftgliedern aus Memory-Metall mit verschlossenem Bypaß-Strömungspfad und
- Figur 8:: eine Ansicht ähnlich Figur 7 mit geöffnetem Bypaß-Strömungspfad.

Die in der Zeichnung dargestellte Filter-Ventil-Anordnung kann grundsätzlich an jeder Wand eines Sterilisierbehälters angeordnet sein, die einen perforierten Bereich 1 aufweist. Im vorliegenden Beispiel ist der perforierte Bereich 1 in der oberen Wand 2 eines in der Zeichnung nur teilweise dargestellten Deckels angeordnet. Der perforierte Bereich 1 kann beispielsweise eine kreisförmige Fläche einnehmen.

Bei dem dargestellten Ausführungsbeispiel wird dieser perforierte Bereich 1 im Abstand von einer den perforierten Bereich 1 nur über einen Teil seines Umfanges umgebenden Halteleiste 3 umschlossen, die einen ersten, flächig an der Wand 2 anliegenden und in diesem Bereich an der Wand 2 gehaltenen Bereich 4 und einen auf den perforierten Bereich 1 hin gerichteten, gegenüber dem Bereich 4 seitlich versetzten und somit einen Abstand zwischen sich und der Wand 2 einhaltenden zweiten Bereich 5 aufweist. Ein kreiszylindrisches Gehäuse 6 greift mit seinem radial nach außen umgebogenen oberen Rand 7 in den Zwischenraum 8 zwischen dem Bereich 5 der Halteleiste 3 und dar Wand 2, so daß das Gehäuse 6 auf diese Weise an der Wand 2 gehalten ist. Auch der Rand 7 erstreckt sich nur über einen Teil des Umfangs des zylindrischen Gehäuses 6, so daß durch Verdrehen des Gehäuses 6 um die Zylinderachse der Rand 7 aus dem Zwischenraum 8 herausgedreht werden kann, bis das Gehäuse 6 von der Wand 2 abnehmbar ist.

Die der Wand 2 abgewandte untere Stirnwand 9 ist im wesentlichen eben ausgebildet und verschließt das zylindrische Gehäuse 6 weitgehend. Der zentrale Teil der Stirnwand 9 wird begrenzt durch eine Dichtrippe 10. Radial außerhalb der Dichtrippe 10 sind längs des Umfanges der Stirnwand 9 eine größere Anzahl von Öffnungen 11 vorgesehen.

Der perforierte Bereich 1 wird von einem nach unten von der Wand 2 abstehenden, nach unten ragenden Stutzen 12 umgeben, dessen unterer Rand 13 als Dichtkante ausgebildet ist.

Oberhalb der als Abdeckung wirkenden Stirmwamd 9 ist eine Filterscheibe 14 angeordnet, die mit ihrem äußeren, nach oben gezogenen Rand 15 den Stutzen 12 an der Außenseite umfängt und zusammen mit dem Stutzen 12 eine Gleitführung für die Filterscheibe 14 bildet, die es ermöglicht, die Filterscheibe in senkrechter Richtung gegenüber der Stirnwand 9 zu verschieben. Seitlich sind in dem nach oben umgebogenen Rand 15 der Filterscheibe 14 Durchströmöffnungen 16 vorgesehen, die sich in einem Bereich befinden, in dem der Stutzen 12 nach inner abgekröpft ist, so daß der Stutzen 12 von dem Rand 13 einen geringen radialen Abstand aufweist.

Die Filterscheibe 14 trägt im zentralen Bereich ein ringförmiges Filter 17, welches sich in radialer Richtung etwa bis zum Beginn der Dichtrippe 10 in der Stirnwand 9 erstreckt und welches im zentralen Bereich der Filterscheibe 14 bis zu einer zentralen Nabe 40 reicht. Das Filter kann ein Metallsieb oder ein Glasfasersieb sein, es kann auch aus Kunststoff-Fasern bestehen.

Der äußere Rand des Filters 17 wird umgeben von zwei nebeneinanderliegenden Ringnuten 18 und 19, die durch Einprägung in der Filterscheibe 14 gebildet sind. Die innere Ringnut 10 öffnet sich nach unten und befindet sich oberhalb der Dichtrippe 10 in der Stirwand 9. Die äußere Ringnut 19 schließt sich unmittelbar an die innere Ringnut 18 an und ist nach oben hin geöffnet. In beiden Ringnuten 18 und 19 ist jeweils eine Silikondichtung 20 beziehungsweise 21 eingelegt. Unmittelbar oberhalb der nach unten offenen Ringnut 18 ist an der Filterscheibe 14 eine umlaufende Dichtkante 22 angeordnet.

Eine Schraubenfeder 23 ist zwischen Stirnwand 9 und Filterscheibe 14 angeordnet, die sich mit ihrem unteren Teil an der Stirnwand 9 und mit dem oberen Teil an der Nabe 40 abstützt. Diese Schraubenfeder 23 verschiebt die Filterscheibe 14 in Richtung auf die Wand 2, bis die Silikondichtung 21 in der äußeren Ringnut 19 am unteren Rand 13 des Stutzens 12 anliegt.

In dem von dem Stutzen 12 umschlossenen Raum befindet sich eine Abdeckscheibe 27, die in ihrem zentralen Bereich eine nach unten abstehende Ringwand 26 trägt. Diese Ringwand 26 umschließt die Nabe 40 in der Filterscheibe 14 und wird dadurch in senkrechter Richtung verschieblich an der Filterscheibe 14 gelagert. Die Abdeckscheibe 27 ist im wesentlichen eben und ohne Öffnungen ausgebildet, sie trägt an ihrem äußeren Rand eine nach unten offene Ringnut 30, die sich genau über der inneren Ringnut 18 der Filterscheibe 14 befindet und ebenfalls eine Silikondichtung 31 aufnimmt. Zwischen der Filterscheibe 14 und der Abdeckscheibe 27 ist eine weitere Schraubenfeder 32 angeordnet, die sich einerseits an der Nabe 40 und andererseits an der Unterseite der Abdeckscheibe 27 abstützt und dadurch die Abdeckscheibe von der Filterscheibe zu entfernen sucht.

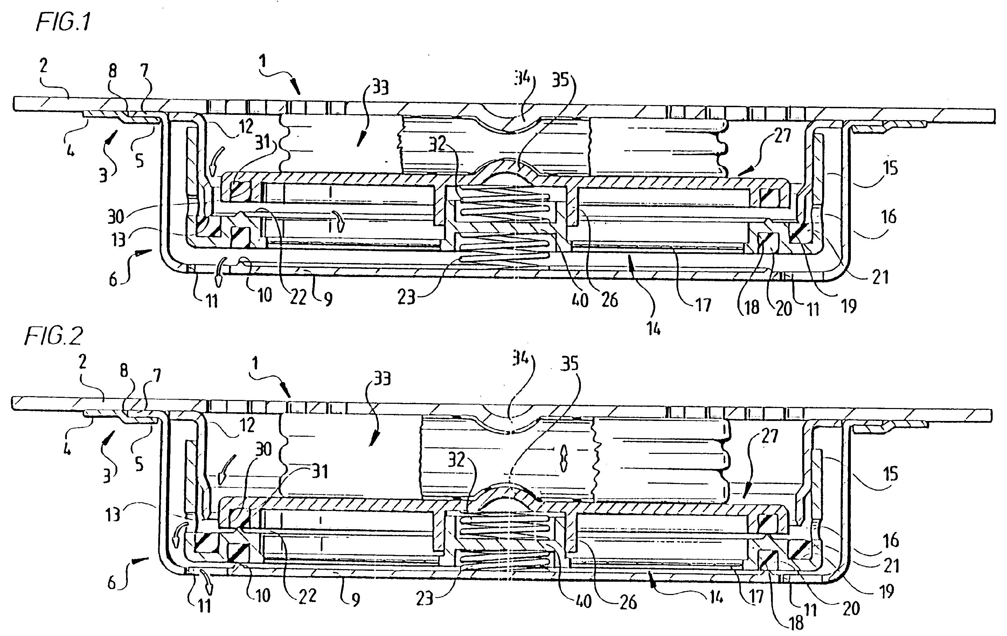

Zwischen dem perforierten Bereich 1 und der Oberseite der Abdeckscheibe 27 ist bei dem Ausführungsbeispiel der Figuren 1 und 2 ein weiteres Kraftglied 33 in Form eines Balges angeordnet, der sich flächig an den perforierten Bereich 1 und die Oberseite der Abdeckscheibe 27 anlegt und in seiner Position durch Ausbiegungen 34 und 35 in der Wand 2 beziehungsweise der Abdeckscheibe 27 fixiert ist. Dieses balgförmige Kraftglied 33 ist mit einem Medium gefüllt, das bei Umgebungstemperatur vorzugsweise flüssig ist -und das bei Erhöhung der Temperatur verdampft, so daß bei Temperaturerhöhung der Druck im Inneren des balgförmigen Kraftgliedes stark zunimmt. Durch diesen Druckanstieg versucht sich das balgförmige Kraftglied auszudehnen, das heißt es übt eine mit der Temperatur ansteigende Kraft auf die Abdeckscheibe 27 aus, die die Abdeckscheibe 27 gegen die Filterscheibe 14 und gegen die Stirnwand 9 zu verschieben sucht.

Selbstverständlich kann das balgförmige Kraftglied 33 auch mit einem Medium gefüllt sein, das bereits bei Umgebungstemperatur gasförmig ist, auch in diesem Falle wird bei einem Temperaturanstieg ein erheblicher Druckansteig und somit eine Tendenz des Kraftgliedes auftreten, mit zunehmender Temperatur eine erhöhte Verschiebekraft auf die Abdeckscheibe 27 auszuüben.

Im normalen Betrieb, wenn also der Sterilisierbehälter bei Umgebungstemperatur betrieben wird und wenn kein starker Gasstrom durch den perforierten Bereich 1 hindurchtritt (Figur 1), ist die Filterscheibe 14 durch die untere Schraubenfeder 23 abdichtend gegen den unteren Rand 13 des Stutzens 12 und die Abdeckscheibe 27 durch die obere Schraubenfeder 32 nach oben gegen das Kraftglied 33 gedrückt. Es ergibt sich dadurch ein Strömungspfad durch die Öffnungen des perforierten Bereiches 1, an der Außenseite der Abdeckscheibe 27 vorbei in den Zwischenraum zwischen Abdeckscheibe 27 und Filterscheibe 14. Der Gasstrom muß in diesem Fall durch das Filter 17 der Filterscheibe 14 hindurchtreten, da die Filterscheibe 14 abdichtend gegen den Stutzen 12 gedrückt ist. Nach dem Durchtritt durch das Filter tritt die Gasströmung durch die Öffnungen 11 in das Behälterinnere ein. Beim Abkühlen des Behälterinhalts, bei dem im Inneren ein Unterdruck entsteht, wird auf diese Weise Luft aus der Umgebung angesaugt, die durch das Filter 17 sicher gereinigt wird. Auch nach dem Sterilisieren noch im Behälterinneren enthaltener Wasserdampf kann auf umgekehrtem Wege durch das Filter 17 nach außen gelangen, ohne daß dabei die Gefahr besteht, daß eine Verunreinigung in das Behälterinnere gelangt.

Wenn dagegen die Temperatur erheblich ansteigt oder wenn eine sehr große Druckdifferenz zwischen außen und innen auftritt und dadurch eine sehr starke nach innen gerichtete Strömung entsteht, -wie dies beispielsweise beim-Eintreten des Wasserdampfes zu Beginn des Sterilisiervorganges der Fall ist, wird dadurch die Abdeckscheibe 27 in Richtung auf die Stirnwand 9 verschoben, bis ihre Silikondichtung 31 abdichtend an der Dichtkante 22 der Filterscheibe 14 anliegt und so das Filter 17 auf der oberen Seite dicht abdeckt. Gleichzeitig drückt die Abdeckscheibe 27 die Filterscheibe 14 bei einem weiteren Verschieben in Richtung auf die Stirnwand 9 gegen den äußeren Teil der Dichtrippe 10, die somit an der Silikondichtung 20 dichtend zur Anlage gelangt und damit das Filter 17 von der Unterseite her dicht abschließt (Figur 2). In dieser Stellung wird das Filter also durch die Abdeckscheibe 27 einerseits und durch die als Abdeckung wirkende Stirnwand 9 andererseits in einem abgedichteten Raum eingeschlossen, so daß das Filter nicht mehr von der Gasströmung durchströmt wird. Das eintretende Gas gelangt vielmehr außen an der Abdeckscheibe 27 und durch die Durchströmöffnungen 16 hindurch unmittelbar zu den Öffnungen 11 in der Stirnwand 9.

Die Verschiebung der Abdeckscheibe 27 und der Filterscheibe 14 wird unterstützt durch die mit dem Temperaturanstieg ansteigende Andruckkraft des Kraftgliedes 33. Es ist dabei möglich, durch geeignete Dimensionierung die Wirkung der temperaturabhängig ansteigenden Verschiebekraft durch das Kraftglied 33 einerseits und die durch die Gasströmung verursachten Verschiebekräfte andererseits so aufeinander abzustimmen, daß je nach Wunsch entweder bei erhöhter Temperatur immer eine Verschiebung entsprechend Figur 2 eintritt oder nur in Unterstützung mit einer bestimmten durch eine Gasströmung hervorgerufenen Druckdifferenz.

Dabei ist bei der Verwendung eines Kraftgliedes mit einer bei Umgebungstemperatur flüssigen Füllung zusätzlich von Vorteil, daß beim Übergang in die Dampfphase ein sehr starker Druckanstieg bei einer bestimmten Temperatur eintritt, so daß es dadurch möglich wird, die Verschiebekräfte unterhalb einer bestimmten Temperatur relativ niedrig zu halten, sie jedoch beim Überschreiten einer bestimmten Temperatur in einem kleinen Temperaturbereich sehr stark ansteigen zu lassen.

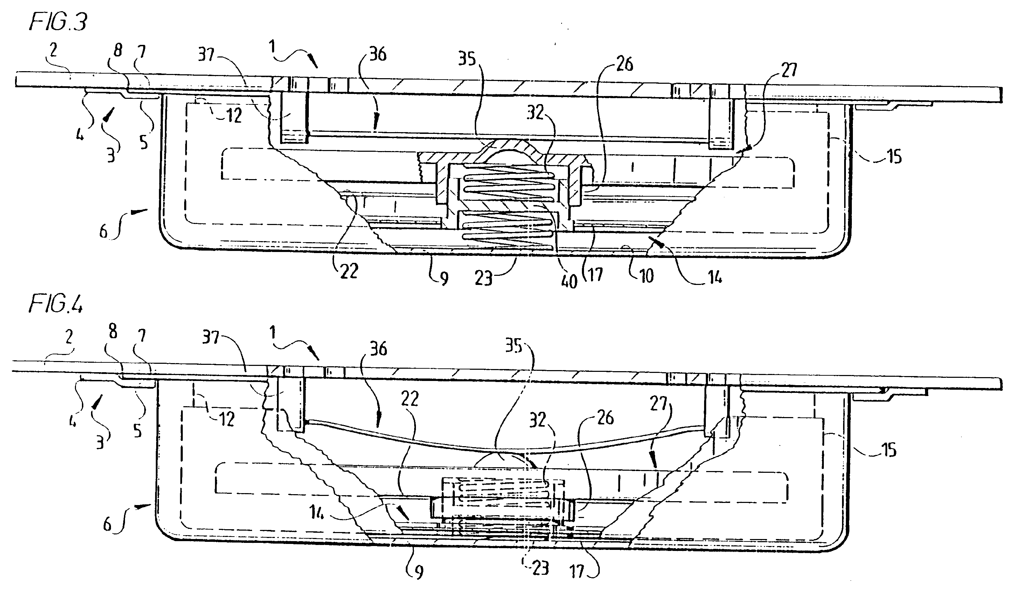

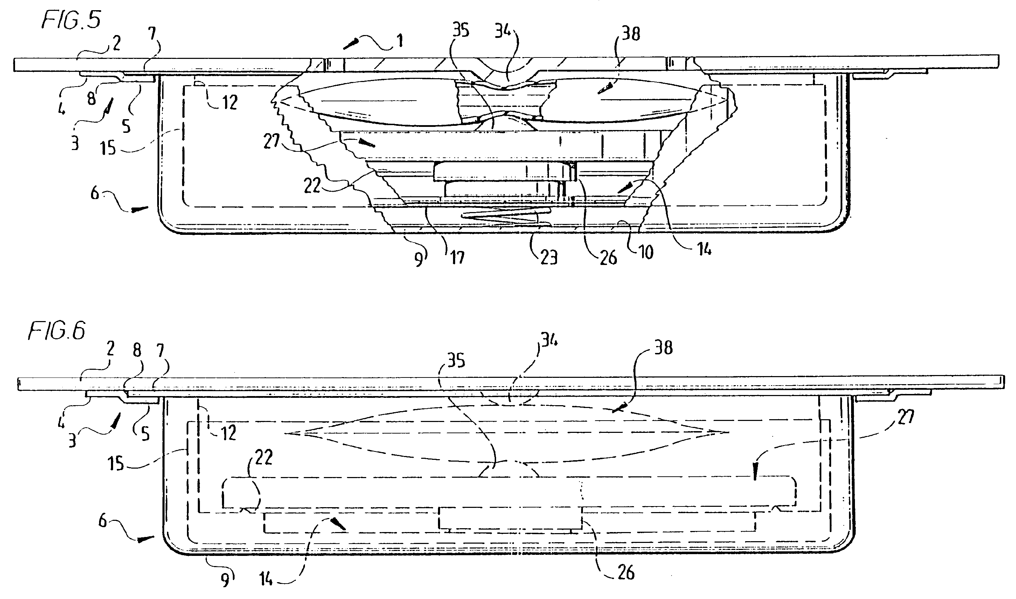

Das in den Figuren 3 und 4 dargestellte Ausführungsbeispiel entspricht weitgehend dem der Figuren 1 und 2, entsprechende Teile tragen daher dieselben Bezugszeichen.

Im Unterschied zu den Ausführungsbeispielen der Figuren 1 und 2 ist das als Balg ausgebildete Kraftglied 33 bei dem Ausführungsbeispiel der Figuren 3 und 4 ersetzt durch ein streifenförmiges Kraftglied 36, welches sich zwischen zwei Stützen 37 erstreckt und welches unmittelbar an der Ausbiegung 35 der Abdeckscheibe 27 anliegt. Das streifenförmige Kraftglied 36 besteht in diesem Ausführungsbeispiel aus einer Memory-Metallegierung, die ein temperaturabhängiges Formgedächtnis hat. Bei niedrigen Temperaturen erstreckt sich dieses Kraftglied 36 zwischen den beiden Stützen 37 im wesentlichen parallel zur Abdeckscheibe 27, bei erhöhten Temperaturen dagegen biegt sich dieses Kraftglied 36 in der in Figur 4 dargestellten Weise in Richtung auf die Abdeckscheibe 27 nach unten aus und beaufschlagt dabei die Abdeckscheibe 27 mit einer zusätzlichen, der Schraubenfeder 32 entgegenwirkenden Kraft. Diese Formänderung des Kraftgliedes 36 ist temperaturabhängig und reversibel, so daß dieses Kraftglied 36 in ähnlicher Weise wie das balgförmige Kraftglied 33 temperaturabhängig eine Zunahme beziehungsweise eine Abnahme der auf die Abdeckscheibe 27 wirkenden Verschiebekräfte bewirkt. Dabei kann das Kraftglied 36 so ausgebildet werden, daß beim Erreichen einer bestimmten Temperatur durch die Formänderung ein Kippeffekt auftritt, daß also das Kraftglied 36 von einer ersten in eine zweite Lage schnappt und dadurch beim Überschreiten dieser Temperatur sprunghaft eine Änderung der auf die Abdeckscheibe 27 wirkenden Verschiebekräfte hervorruft. Diese sprunghafte Änderung der Verschiebekräfte kann beim Anstieg der Temperatur und beim Abfallen der Temperatur bei unterschiedlichen Temperaturen auftreten, das heißt es kann zusätzlich ein Hysterese-Effekt genutzt werden.

Das in den Figuren 5 und 6 dargestellte Ausführungsbeispiel, bei dem wieder entsprechende Teile dieselben Bezugszeichen tragen, entspricht weitgehend dem der Figuren 1 und 2. Das dort dargestellte balgförmige Kraftglied 33 ist bei dem Ausführungsbeispiel der Figuren 5 und 6 durch ein kissenförmiges Kraftglied 38 ersetzt, welches in ähnlicher Weise wie das balgförmige Kraftglied 33 in seiner Lage fixiert wird und welches ebenso mit einem Medium gefüllt ist, welches temperaturabhängig den Druck im Inneren das verformbaren Kissens stark verändert. Die Funktion entspricht somit ebenfalls dem der Ausführungsbeispiele der Figuren 1 und 2.

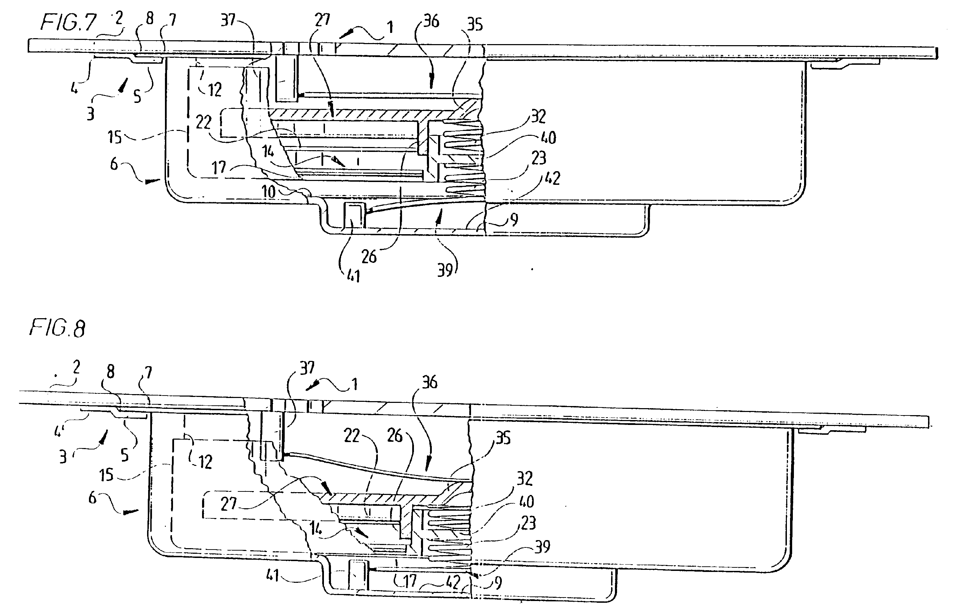

Bei dem Ausführungsbeispiel der Figuren 7 und 8, welches dem der Figuren 3 und 4 weitgehend entspricht und bei dem entsprechende Teile dieselben Bezugszeichen tragen, stützt sich die untere schraubenfeder 23 nicht unmittelbar auf der Stirnwand 9 ab, sondern an einem weiteren Kraftglied 39, welches ebenso wie das Kraftglied 36 aus einer Memory-Metallegierung gebildet ist. Dieses Kraftglied 39 ist zwischen zwei Stützen 41 gehalten, die sich ihrerseits in einem vertieften zentralen Bereich 42 der Stirnwand 9 befinden. Im Gegensatz zu den Kraftglied 36 ist dieses Kraftglied 39 bei niedriger Temperatur ausgebogen, und zwar in Richtung auf die obere Wand 2, so daß bei neidrigen Temperaturen die von der unteren Schraubenfeder 23 auf die Filterscheibe 14 ausgeübte Andruckkraft erhöht wird. Bei Temperaturanstieg geht das Kraftglied 39 in eine Stellung über, in der es sich im wesentlichen parallel zur Filterscheibe 14 erstreckt, so daß dadurch der untere Stützpunkt der Schraubenfeder 23 von der Filterscheibe 14 entfernt wird, das heißt die nach oben gerichtete Verschiebekraft auf die Filterscheibe 14 wird herabgesetzt.

Dadurch wird erreicht, daß bei niedrigen Temperaturen die Filterscheibe 14 mit größerer Kraft gegen den unteren Rand 13 des Stutzens 12 gedrückt wird, so daß eine Erhöhung der Zuverlässigkeit der Abdeckung in diesem Bereich eintritt. Wird die Temperatur erhöht, wird diese zusätzliche Anpreßkraft durch die Verformung des Kraftgliedes 39 aufgehoben, so daß eine Verschiebung der Filterscheibe 14 gegen die Stirnwand 9 erleichtert wird.

Durch die Verwendung von zwei temperaturabhängigen Kraftgliedern ergibt sich einerseits eine besonders zuverlässige Abdichtung bei Umgebungstemperaturen, das heißt im Nichtsterilisationsbetrieb, andererseits wird die Öffnung des Bypasses und die sichere Abdeckung des Filters insbesondere zu Beginn des Sterilisiervorganges erheblich erleichtert.

Selbstverständlich können bei den Ausführungsbeispielen der Figuren 3 und 4 beziehungsweise 7 und 8 die Kraftglieder auch als Bimetall-Elemente ausgebildet werden, ebenso wäre es möglich, bei dem Ausführungsbeispiel der Figuren 7 und 8 die unteren Kraftglieder 39 durch balg- oder kissenförmige Kraftglieder zu ersetzen, wobei diese dann aber über eine Kraftumlenkung wirksam werden müßten, da sie bei niedrigen Temperaturen die Kraft der Schraubenfeder 23 unterstützen, bei hohen Temperaturen jedoch möglichst wenig wirksam sein müssen.

## Patentansprüche

1. Sterilisierbehälter mit einem perforierten Bereich, der durch ein Filter gegenüber dem behälterinnenraum abdeckbar ist,
bei dem zwischen dem Filter und dem perforierten Bereich eine das gesamte Filter überdeckende Abdeckscheibe parallel zur Verschieberichtung des Filters verschieblich gelagert ist,
bei dem eine das Filter und die Abdeckscheibe voneinander entfernende Feder vorgesehen ist,
bei dem Filter und Abdeckscheibe in ihrer am stärksten angenäherten Lage abgedichtet aneinanderliegen,
bei dem an der Unterseite eine behälterfeste Abdeckung angeordnet ist, die das Filter überdeckt,
bei dem eine das Filter von der Abdeckung entfernende Feder vorgesehen ist, gegen deren Wirkung das Filter an die Abdeckung abgedichtet anlegbar ist,
bei dem in der Abdeckung außerhalb des Dichtungsbereiches, innerhalb dessen das Filter abgedeckt ist, Durchströmöffnungen angeordnet sind, die von dem Filter dicht verschlossen werden, wenn dieses von der Abdeckung entfernt ist,
dadurch gekennzeichnet, daß die die Abdeckscheibe (27) und das Filter (14) mit Verschiebekräften beaufschlagenden Kraftglieder (33, 36, 38, 39) die auf die Abdeckscheibe (27) beziehungsweise das Filter (14) ausgeübten Verschiebekräfte in Abhängigkeit von der Temperatur ändern.

2. Sterilisierbehälter nach Anspruch 1, dadurch gekennzeichnet, daß die die Abdeckscheibe (27) gegen das Filter (14) drückenden Kraftglieder (33, 36, 38) die Andruckkräfte mit steigender Temperatur erhöhen.

3. Sterilisierbehälter nach Anspruch 1 oder 2, dadurch gekennzeichnet, daß die das Filter (14) gegen die Abdeckung (27) drückenden Kraftglieder (39) die Andruckkräfte mit steigender Temperatur herabsetzen.

4. Sterilisierbehälter nach einem der veranstehenden Ansprüche, dadurch gekennzeichnet, der die Kraftglieder (33, 38) abgeschlossene Körper die verformbaren Wänden sind, die ein Medium enthalten, das sich mit steigender Temperatur ausdehnt.

5. Sterilisierbehälter nach Anspruch 4, dadurch gekennzeichnet, daß die Körper mit einer Flüssigkeit gefüllt sind, die bei zunehmender Temperatur verdampft.

6. Sterilisierbehälter nach Anspruch 4 oder 5, dadurch gekennzeichnet, daß die Körper kissenförmig ausgebildet sind.

7. Sterilisierbehälter nach Anspruch 4 oder 5, dadurch gekennzeichnet, daß die Körper balgförmig ausgebildet sind.

8. Sterilisierbehälter nach einem der Ansprüche 1 bis 3, dadurch gekennzeichnet, daß die Kraftglieder (36, 39) aus Memory-Metall bestehen.

9. Sterilisierbehälter nach einem der Ansprüche 1 bis 3, dadurch gekennzeichnet, daß die Kraftglieder (36, 39) als Bimetall-Elemente ausgebildet sind.

10. Sterilisierbehälter nach einem der voranstehenden Ansprüche, dadurch gekennzeichnet, daß ein Kraftglied (33, 36, 38) zwischen dem perforierten Bereich (1) und der Abdeckscheibe (27) angeordnet ist.

11. Sterilisierbehälter nach einem der voranstehenden Ansprüche, dadurch gekennzeichnet, daß ein Kraftglied (39) zwischen Abdeckung (9) und Filter (14) angeordnet ist.

12. Sterilisierbehälter nach Anspruch 11, dadurch gekennzeichnet, daß sich an dem Kraftglied (39) zwischen Abdeckung (9) und Filter (14) die Feder (23) abstützt, die das Filter (14) von der Abdeckung (9) entfernt.

## Claims

1. Sterilizing container with a perforated region adapted to be covered by a filter relative to the interior of the container,
wherein a cover plate covering the entire filter is mounted between the Filter and the perforated region for displacement parallel to the direction of displacement of the filter,
wherein a spring is provided for moving the filter and the cover plate apart from one another,
wherein filter and cover plate contact one another in a sealing manner in their position of closest proximity, wherein a cover fixed to the container is arranged on the underside thereof and covers the filter,
wherein a spring is provided for moving the filter away from the cover, the filter being adapted to be applied in a sealing manner to the cover contrary to the action of this spring,
wherein flow openings are arranged in the cover outside the sealed region within which the filter is covered, these flow openings being sealingly closed by the filter when the filter is moved away from the cover,
characterized in that the force members (33, 36, 38 ,39) acting with displacing forces on the cover plate (27) and the filter (14) alter the displacing forces exerted on the cover plate (27) and the filter (14), respectively, as a function of the temperature.

2. Sterilizing container as defined in claim 1, characterized in that the force members (33, 36, 38) pressing the cover plate (27) against the filter (14) increase the pressing forces as the temperature rises.

3. Sterilizing container as defined in claim 1 or 2, characterized in that the force members (39) pressing the filter (14) against the cover (27) reduce the pressing forces as the temperature rises.

4. Sterilizing container as defined in any of the preceding claims, characterized in that the force members (33, 38) are closed bodies having deformable walls containing a medium expanding as the temperature rises.

5. Sterilizing container as defined in claim 4, characterized in that the bodies are filled with a liquid evaporating as the temperature rises.

6. Sterilizing container as defined in claim 4 or 5, characterized in that the bodies are of a cushion-shaped design.

7. Sterilizing container as defined in claim 4 or 5, characterized in that the bodies are of a bellows-type design.

8. Sterilizing container as defined in any of claims 1 to 3, characterized in that the force members (36, 39) consist of memory metal.

9. Sterilizing container as defined in any of claims 1 to 3, characterized in that the force members (36, 39) are designed as bimetal elements.

10. Sterilizing container as defined in any of the preceding claims, characterized in that one force member (33, 36, 38) is arranged between the perforated region (1) and the cover plate (27).

11. Sterilizing container as defined in any of the preceding claims, characterized in that one force member (39) is arranged between cover (9) and filter (14).

12. Sterilizing container as defined in claim 11, characterized in that the spring (23) moving the filter (14) away from the cover (9) is supported on the force member (39) between cover (9) and filter (14).

## Revendications

1. Récipient de stérilisation comprenant une région perforée qui peut être isolée du volume intérieur du récipient par un filtre,
dans lequel il est prévu, entre le filtre et la région perforée, un disque de recouvrement qui recouvre tout le filtre et qui est monté pour pouvoir se déplacer parallèlement à la direction de translation du filtre,
dans lequel il est prévu un ressort qui tend à éloigner le filtre et le disque de recouvrement l'un de l'autre,
dans lequel le filtre et le disque de recouvrement sont appuyés à joint étanche dans leur position la plus fortement rapprochée ;
dans lequel il est prévu, contre la face inférieure, un recouvrement solidaire du récipient qui recouvre le filtre,
dans lequel il est prévu un ressort qui tend à éloigner le filtre du recouvrement et à l'encontre de l'action duquel le filtre peut être appliqué à joint étanche contre le recouvrement,
dans lequel il est prévu dans le recouvrement, en dehors de la région étanche à l'intérieur de laquelle le filtre est recouvert, des ouvertures de traversée qui sont fermées à joint étanche par le filtre lorsque celui-ci est éloigné du recouvrement,
caractérisé en ce que les générateurs de force (33, 26, 38, 39) qui attaquent le disque de recouvrement (27) et le filtre (14) par des forces de translation, font varier en fonction de la température les forces de translation exercées sur le disque de recouvrement (27) ou sur le filtre (14) respectivement.

2. Récipient de stérilisation selon la revendication 1, caractérisé en ce que les générateurs de force (33, 36, 38) qui repoussent le disque de recouvrement (27) vers le filtre (14) font croître les forces de pression avec l'élévation de la température.

3. Récipient de stérilisation selon la revendication 1 ou 2, caractérisé en ce que les générateurs de force (39) qui repoussent le filtre (14) vers le recouvrement (27) font décroître les forces de pression avec l'élévation de la température.

4. Récipient de stérilisation selon une des revendications précédentes, caractérisé en ce que les générateurs de force (33, 38) sont des corps fermés à paroi déformable qui contiennent un fluide qui se dilate avec l'élévation de la température.

5. Récipient de stérilisation selon la revendication 4, caractérisé en ce que les corps sont remplis d'un liquide qui se vaporise avec l'élévation de la température.

6. Récipient de stérilisation selon la revendication 4 ou 5, caractérisé en ce que les corps sont en forme de coussin.

7. Récipient de stérilisation selon la revendication 4 ou 5, caractérisé en ce que les corps sont en forme de soufflet.

8. Récipient de stérilisation selon une des revendications 1 à 3, caractérisé en ce que les générateurs de force (36, 39) sont constitués par un métal à mémoire.

9. Récipient de stérilisation selon une des revendications 1 à 3, caractérisé en ce que les générateurs de force (36, 39) sont constitués par des éléments bimétalliques.

10. Récipient de stérilisation selon une des revendications précédentes, caractérisé en ce qu'un générateur de force (33, 36, 38) est interposé entre la région perforée (1) et le disque de recouvrement (27).

11. Récipient de stérilisation selon une des revendications précédentes, caractérisé en ce qu'un générateur de force (39) est interposé entre le recouvrement (9) et le filtre (14).

12. Récipient de stérilisation selon la revendication 11, caractérisé en ce que le ressort (23) qui tend à éloigner le filtre (14) du recouvrement (9) prend appui sur le générateur de force (39) entre le recouvrement (9) et le filtre (14).
